# EUROPÄISCHE PATENTSCHRIFT

(11) **EP 1 140 987 B1**
(45) Veröffentlichungstag und Bekanntmachung des Hinweises auf die Patenterteilung: **14.05.2003**
(21) Anmeldenummer: 00908931.9
(22) Anmeldetag: 12.01.2000
(51) Int. Cl.: C07K 7/02, C07K 14/025, A61K 38/10, C07K 14/47

(54) **PEPTIDE ZUR INHIBIERUNG VON HPV E6-PROTEINEN**
PEPTIDES FOR INHIBITING HPV E6 PROTEINS
PEPTIDE POUR L'INHIBITION DES PROTEINES E6 DU VIRUS DE L'HEPATITE B

(30) Priorität: 13.01.1999 DE 19901008
(43) Veröffentlichungstag der Anmeldung: 10.10.2001
(73) Patentinhaber: DEUTSCHES KREBSFORSCHUNGSZENTRUM STIFTUNG DES ÖFFENTLICHEN RECHTS, 69120 Heidelberg (DE)
(72) Erfinder: BUTZ, Karin, D-69493 Hirschberg (DE); HOPPE-SEYLER, Felix, D-69118 Heidelberg (DE)
(74) Vertreter: Schüssler, Andrea, Dr.
(86) Internationale Anmeldenummer: DE0000141
(87) Internationale Veröffentlichungsnummer: WO00042064

(56) Entgegenhaltungen:
- WO-A-96/02000
- WO-A-97/18309
- US-A- 5 532 348

## Beschreibung

Die vorliegende Erfindung betrifft Peptide, die sich zur Inhibierung von HPV E6-Proteinen eignen, sie kodierende DNAs und die Verwendung beider, insbesondere zur Eliminierung von HPV positiven Zellen, z.B. HPV-Tumorzellen.

Humane Papillomviren (HPVs) sind eng mit der Entwicklung von Karzinomen verbunden. Gut charakterisiert ist die Beteiligung von HPVs an der Entstehung des Zervixkarzinoms und verschiedene Befunde weisen darauf hin, daß HPVs eine kausale Rolle in der Ätiologie dieses Karzinoms spielen. Auf der molekularen Ebene sind in etwa 95 % der Zervixkarzinom-Biopsien Sequenzen von HPVs, insbesondere HPV 16 und 18, nachweisbar. Die HPV-DNA liegt dabei meist integriert im Genom der Tumorzellen vor. Sie weist häufig Deletionen und/oder Rearrangements auf, wobei sich diese nie auf die frühen HPV-Gene, nämlich die E6- und E7-Gene, beziehen. Diese Gene kodieren für die HPV-Proteine E6 und E7, die für die Entstehung und Manifestierung von HPV-Karzinomen verantwortlich sind. Die HPV-Proteine wirken dabei synergistisch. Andererseits gibt es Hinweise, daß sie auch entgegengesetzte Aktivitäten aufweisen. Beispielsweise induziert das E7-Protein Apoptose, während das E6-Protein eine solche inhibiert. Dies erfolgt dadurch, daß das E6-Protein direkt oder indirekt, d.h. über Ubiquitin-Protein-Ligase E6-AP, an p53 bindet und dieses hemmt, wodurch p53 seine Apoptose-induzierende Aktivität nicht mehr entfalten kann.. Desweiteren besitzt das E6-Protein auch eine p53 unabhängige anti-apoptotische Aktivität. Die Gene der E6- und E7-Proteine werden über polycistronische mRNAs exprimiert, wobei die Transkription unter der Kontrolle eines gemeinsamen Promotors steht. Es sind Versuche bekannt, letzteren bzw. E6-/E7 mRNAs zu inhibieren. Diese Versuche führten zu einer partiellen Inhibierung des Wachstums von HPV-Tumorzellen. Eine Eliminierung solcher Zellen, was sehr erwünscht ist, konnte damit aber nicht erreicht werden.

Aus WO 97/18309 und WO 96/02000 sind jeweils Polypeptid-Bindungsproteine von HPV-E6 bekannt, die agonistisch bzw. antagonistisch zu HPV-E6 wirken. Ferner sind aus US 5 532 348 assoziierte Proteine von HPV-E6 bekannt, die am Komplex aus HPV-E6 und p53 beteiligt sind.

Der vorliegenden Erfindung liegt somit die Aufgabe zugrunde, ein Mittel bereitzustellen, mit dem HPV-Tumorzellen, eliminiert werden können.

Erfindungsgemäß wird dies durch die Gegenstände in den Patentansprüchen erreicht.

Der Anmelder hat erkannt, daß HPV E6-Proteine an kurze Peptide binden. Er hat eine randomisierte Oligopeptid-Bibliothek, die zufallsgenerierte Peptid-Sequenzen umfaßt, mit einem "Peptid-Aptamer-System" gescreent, in dem das HPV-E6-Protein als Screening-Probe verwendet wurde. Hierbei hat er gefunden, daß kurze Peptide, insbesondere die in Tabelle 1 aufgeführten, HPV E6-Proteine binden. Ferner hat er erkannt, daß sich diese Peptide eignen, Aktivitäten von HPV E6-Proteinen, z.B. ihre anti-apoptotische Aktivität, zu inhibieren. Des weiteren hat er erkannt, daß durch diese Inhibierung eine Eliminierung von HPV-positiven Zellen, insbesondere HPV-Tumorzellen, erreicht werden kann.

Erfindungsgemäß werden die Erkenntnisse des Anmelders genutzt, ein Peptid bereitzustellen, das aus den in Tabelle 1 aufgelisteten Peptiden ausgewählt ist, wobei das Peptid eine Modifikation in der Sequenz von bis zu 40 %, insbesondere 20 % und ganz besonders 10 %, aufweisen kann.

**Tabelle 1**

| | |
|---|---|
| E61-1.pep | NH₂-GALVHKLFSQ TSGSCLVCIS-COOH |
| E61-2.pep | NH₂-LDVLGCLVRR LGVVLVGLH-COOH |
| E61-3.pep | NH₂-CYVECGCEVL TALVNGVRVL-COOH |
| E61-5.pep | NH₂-GVGGLCSCAS CVSEDFYASV-COOH |
| E61-7.pep | NH₂-IDLLRRLSQ LHLLLVSVGG-COOH |
| E61-8.pep | NH₂-LAVLLNGYTRAIVGISFGGW-COOH |
| E61-9c.pep | NH₂-LCTMCATVFR PLLVWFWSIW-COOH |
| E61-10.pep | NH₂-QLLLDLLLGS YEGMSLTSSP-COOH |
| E61-11.pep | NH₂-SRSNALHTLD VLLGGT-COOH |
| E61-12.pep | NH₂-GGAVYLCDAG CCFYCCGCSG-COOH |
| E61-13.pep | NH₂-CLELFDDLFL ALSLLLLVGG-COOH |
| E61-14.pep | NH₂-PLCRTCLIES AVLIQLSRL-COOH |
| E61-15.pep | NH₂-VFSGVYYAEF VFAASAGGTP-COOH |
| E61-16.pep | NH₂-MAPVGAGRPC CTVCFLTARF-COOH |
| E61-17.pep | NH₂-LSMLLFAAKL PVAVLCSWQA-COOH |
| E61-19.pep | NH₂-LVGRVRIGVS VFIRGGRLL-COOH |
| E61-20.pep | NH₂-LFDIFRLCAQPVLVHGHTRV-COOH |

Erfindungsgemäße Peptide eignen sich HPV E6-Proteine zu binden und in ihren Aktivitäten, z.B. in ihrer anti-apoptotischen Aktivität, zu inhibieren.

Der Ausdruck "HPV E6-Proteine" umfaßt ein E6-Protein jeglichen HPV-Typs, insbesondere von HPV1, 5, 6, 11, 16, 18, 31, 33 oder 35. Ein E6-Protein kann eine Wildtyp-Sequenz oder eine hiervon durch ein oder mehrere Aminosäuren unterschiedliche Sequenz aufweisen. Ferner kann es in verkürzter Form vorliegen, d.h. es liegt nur in Form jenes Fragments vor, das für die Bindung an p53, Ubiquitin-Protein-Ligase E6-AP oder einen anderen Bindungspartner des E6-Proteins notwendig ist. Das Fragment kann auch in Mehrfachkopien innerhalb eines Polypeptid-Moleküls vorliegen. Des weiteren kann ein E6-Protein bzw. ein Fragment davon in Form eines Fusionsproteins vorliegen.

Erfindungsgemäße Peptide können durch übliche Verfahren, in denen Peptide hinsichtlich ihrer Bindung an HPV E6-Proteine getestet werden, bereitgestellt werden. Solche Verfahren sind z.B. das "Peptid-Aptamer-" oder "Bakteriophagen-Display"-Verfahren. Günstig ist es, das in den Beispielen beschriebene "Peptid-Aptamer"-Verfahren zu verwenden, das eine Modifizierung des vorstehend erwähnten Verfahrens ist.

Erfindungsgemäße Peptide können als solche oder in Verbindung mit anderen Stoffen, z.B. (Poly) peptiden, vorliegen. Die Verbindung kann darin bestehen, daß die erfindungsgemäßen Peptide über Linker, z.B. Disulfidbrücken, mit den (Poly)peptiden verbunden sind. Auch können die erfindungsgemäßen Peptide mit den (Poly)peptiden fusioniert sein, wodurch die erfindungsgemäßen Peptide in Form von Fusions(poly)peptiden vorliegen. Als (Poly)peptide für Fusions(poly)peptide bieten sich z.B. "Leader"-Peptide, wie Penetratin von Drosophila Antennapedia oder VP22 von HSV1 an, welche die Aufnahme der erfindungsgemäßen Peptide in Zellen fördern. Andererseits können Polypeptide, die über Linker mit dem erfindungsgemäßen Peptid verbunden sind, z.B. Trägerproteine, wie Transferrin sein, die im Körper als nicht fremd angesehen werden. Auch können mehrere erfindungsgemäße Peptide gleichzeitig in Verbindung mit einem vorstehenden Stoff vorliegen.

Ein weiterer Gegenstand der vorliegenden Erfindung ist eine Nukleinsäure, insbesondere eine DNA, die für ein erfindungsgemäßes Peptid kodiert. Eine solche DNA kann in Vektoren, insbesondere Expressionsvektoren vorliegen. Beispiele solcher sind dem Fachmann bekannt. Im Falle eines Expressionsvektors für E. coli sind dies z.B. pGEMEX, pUC-Derivate, pGEX-2T, pET3b oder pQE-8. Für die Expression in Hefe sind z.B. pY100 oder Ycpad1 zu nennnen, während für die Expression in tierischen Zellen z.B. pKCR, pEFBOS, cDM8 oder pCEV4 anzugeben sind. Für die Expression in Insektenzellen eignet sich besonders der Bacculovirus-Expressionsvektor pAcSGHisNT-A. Ferner können für die Expression in tierischen Zellen Viren, z.B. Adenovirus, Vaccinia-Virus, Adeno-assoziierter Virus (AAV) oder Retroviren, wie MoMuLV, HaMuSV, MuMTV, RSV oder GaIV), verwendet werden.

Der Fachmann kennt geeignete Zellen, um die erfindungsgemäße, in einem Expressionsvektor vorliegende DNA zu exprimieren. Beispiele solcher Zellen umfassen die E.coli-Stämme HB101, DH1, x1776, JM101, JM109, BL21 und SG 13009, den Hefe-Stamm Saccharomyces cerevisiae und die tierischen Zellen L, NIH 3T3, FM3A, CHO, COS, Vero und HeLa sowie die Insektenzellen sf9.

Der Fachmann weiß, in welcher Weise die erfindungsgemäße DNA in einen Expressionsvektor inseriert werden muß. Ihm ist auch bekannt, daß diese DNA in Verbindung mit einer für ein anderes Peptid bzw. Polypeptid kodierenden DNA inseriert werden kann, so daß die erfindungsgemäße DNA in Form eines Fusionspolypeptids exprimiert werden kann.

Des weiteren kennt der Fachmann Bedingungen, transformierte bzw. transfizierte Zellen zu kultivieren. Auch sind ihm Verfahren bekannt, das durch die erfindungsgemäße DNA exprimierte Peptid bzw. Fusionspolypeptid zu isolieren und zu reinigen.

Ein weiterer Gegenstand der vorliegenden Erfindung ist ein gegen ein vorstehendes Peptid bzw. Fusionspolypeptid gerichteter Antikörper. Ein solcher Antikörper kann durch übliche Verfahren hergestellt werden. Er kann polyklonal bzw. monoklonal sein. Zu seiner Herstellung ist es günstig, Tiere, insbesondere Kaninchen oder Hühner für einen polyklonalen und Mäuse für einen monoklonalen Antikörper, mit einem vorstehenden (Fusions)polypeptid oder Fragmenten davon zu immunisieren. Weitere "Booster" der Tiere können mit dem gleichen (Fusions)polypeptid oder Fragmenten davon erfolgen. Der polyklonale Antikörper kann dann aus dem Serum bzw. Ei der Tiere erhalten werden. Für den monoklonalen Antikörper werden Milzzellen der Tiere isoliert und mit Myelomzellen fusioniert.

Ein weiterer Gegenstand der vorliegenden Erfindung ist eine pharmazeutische Zusammensetzung, die ein oder mehrere der erfindungsgemäßen Peptide und/oder sie kodierende DNAs sowie übliche Hilfsstoffe enthält. Als Hilfsstoffe können z.B. Arzneimittelträger, Bindemittel, Sprengmittel, Gleitmittel, Lösungsmittel, Lösungsmittelvermittler, Freigabe-Beschleuniger, Freigabe-Verzögerer, Emulgatoren, Stabilisatoren, etc. verwendet werden. Eine solche Zusammensetzung kann in üblicher Weise, z.B. oral oder parenteral, verwendet werden. Die geeignete Dosierung wird für den Einzelfall in üblicher Weise bestimmt.

Ein weiterer Gegenstand der vorliegenden Erfindung ist eine diagnostische Zusammensetzung, die ein oder mehrere erfindungsgemäße Peptide enthält. Mit einer solchen Zusammensetzung können HPV E6-Proteine nachgewiesen werden. Dies kann genutzt werden, um HPV-assoziierte Erkrankungen, wie HPV-Infektionen, HPV-Dysplasien oder HPV-Karzinome, nachzuweisen. Ein solcher Nachweis beinhaltet beispielsweise (a) Gewinnung einer Zellprobe von einem Patienten, (b) Inkontaktbringung der Zellprobe mit einem erfindungsgemäßen Peptid unter Bedingungen, welche die spezifische Bindung des Peptids an ein HPV E6-Protein erlauben, und (c) Nachweis des Peptids. Dieser Nachweis kann durch Standardverfahren erfolgen. Beispielsweise können die erfindungsgemäßen Peptide in Flüssigphase vorliegen oder an einen festen Träger gebunden werden und auf verschiedene Art und Weise markiert sein. Geeignete Marker und Markierungsverfahren sind dem Fachmann bekannt. Auch können die Peptide durch erfindungsgemäße Antikörper nachgewiesen werden. Letztere eignen sich ferner dazu, den Therapieverlauf einer mit erfindungsgemäßen Peptiden behandelten HPV-assoziierten Erkrankung zu kontrollieren.

Mit der vorliegenden Erfindung ist es möglich HPV E6-Proteine zu binden und in ihren Aktivitäten, insbesondere in ihrer anti-apoptotischen Aktivität, zu inhibieren. Damit kann die Eliminierung von HPV-positiven Zellen, insbesondere HPV-Tumorzellen, erreicht werden. Ferner können HPV-positive Zellen diagnostiziert werden. Damit eignet sich die vorliegende Erfindung gegen HPV-assoziierte Erkrankungen, insbesondere HPV-Infektionen, HPV-Dysplasien oder HPV-Karzinome, diagnostisch und therapeutisch vorzugehen. Des weiteren stellen die erfindungsgemäßen Peptide bzw. sie kodierende DNAs eine Basis dar, völlig neue Wirkstoffe gegen vorstehende Erkrankungen zu entwickeln.

### Kurze Beschreibung der Zeichnungen.

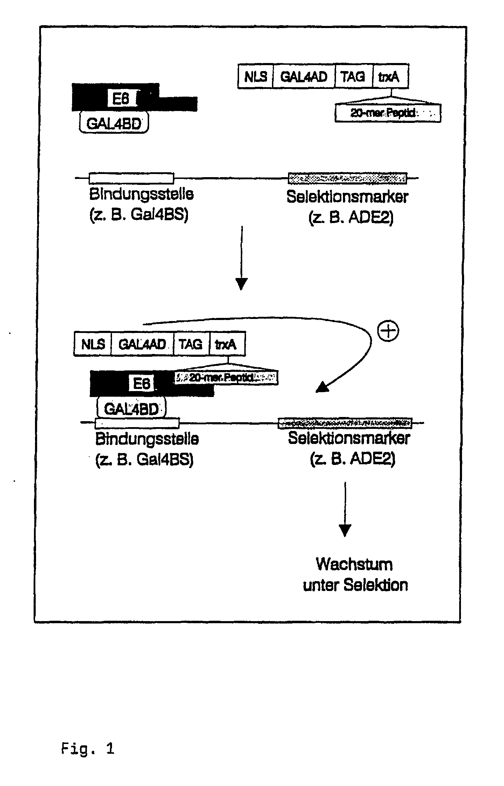

- Fig. 1: zeigt schematisch das modifizierte "Peptid-Aptamer"-System in S. cerevisiae. Dieses System umfaßt drei Komponenten: (1) das Zielprotein (E6), das mit einer heterologen DNA-Bindungsdomäne (GAL4BD) fusioniert ist, (2) ein Peptid mit randomisierter Aminosäuresequenz, das an eine transkriptionelle Aktivierungsdomäne (GAL4AD) fusioniert ist und (3) ein stabil integriertes Selektionsgen (prototrophe Selektionsmarker, wie ADE2), das in seinem Promotor die Erkennungssequenz für die DNA-Bindungsdomäne besitzt. Durch Interaktion zwischen dem Peptid und dem Zielprotein entsteht ein synthetischer Transkriptionsfaktor, der durch die Transaktivierungsdomäne an die Erkennungssequenz im Promotorbereich des Selektionsgens bindet und durch die Transaktivierungsdomäne die Transkription des Selektionsgens stimuliert. Unter Selektionsbedingungen (z.B. Adenin-negativen Nährböden) bilden nur jene Hefezellen Kolonien, die ein Peptid mit Affinität für das Zielprotein exprimieren (TrxA = E.coli Thioredoxin A; TAG = Influenza Virus HA-Tag; NLS = nukleäres Lokalisationssignal).

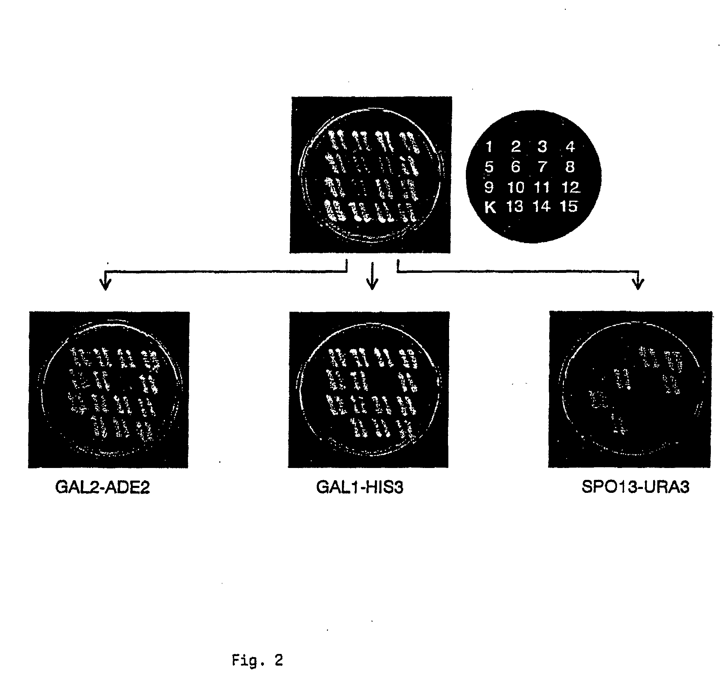

- Fig. 2: zeigt die Analyse von HPV16 E6-bindenden Peptiden im "Peptid-Aptamer"-System. Durch Screening von etwa 2 x 10⁶ Hefezellen werden 15 positive Klone isoliert (vgl. Tabelle 1, E61-1.pept - E61.-17.pep). Es werden Replika-Plattierungen der Hefekolonien (Masterplatte oben: 1-15 = positive Klone; K = zufällig ausgewählter Kontroll-Klon) unter Selektion auf ADE2 (GAL4-BS im Kontext des GAL2-Promotors), HIS3 (GAL4BS im Rahmen des GAL1-Promotors) und URA3 (GAL4-BS im Rahmen des SPO13-Promotors) durchgeführt.
Die folgenden Beispiele erläutern die Erfindung.

### Beispiel 1: Screening nach HPV E6-Protein bindenden Peptiden

Es wird ein Verfahren verwendet, das sich von dem bekannten "Peptid-Aptamer"-System ableitet. Das Verfahren ist in Fig. 1 schematisch dargestellt.
Für das Screening nach HPV E6-Protein bindenden Peptiden wird eine randomisierte Oligopeptid-Expressionsbank für 20 Aminosäuren-lange Peptide mit zufälliger Sequenz hergestellt (Komplexität ca. 2 x 10⁸ unterschiedliche Peptide). Kodons werden durch die Sequenz NNK definiert (N = G, A, T oder C; K = G oder C). Sie kodieren für alle 20 Aminosäuren, resultieren aber nur in einem Stop-Kodon. Als Expressionsvektor wird ein Hefe-Expressionsvektor, pADTrx, verwendet. Dieser enthält E.coli Trx A (Thioredoxin-Protein) aus pTrx (Invitrogen) und GAL4AD sowie den ADH-Promotor aus pAS2 bzw. pGAD424 (Clontech). Die Peptide werden im Rahmen der aktiven Schleife von Trx exprimiert. Dies hat folgende Vorteile:
- im Rahmen der Trx-Schleife ist die Präsentation der Peptide nach außen gewährleistet,
- konformell restringierte Peptide können Aminosäuren nach außen exponieren, die bei flexiblen Peptiden im intrazellulären Milieu möglicherweise nach innen gefaltet werden,
- konformell restringierte Peptide können hochaffine Peptide sein, die das Potential besitzen, auch in vivo als effiziente Proteininhibitoren zu wirken.

Ferner wird ein Hefestamm, KF-1, verwendet. Dieser stammt von dem Hefestamm PJ69-4A (vgl. James et al., Genetics 144 (1996), 1425) und erlaubt die Analyse von drei Selektionsmarkern: ADE2, HIS3 und URA3. Jeder der drei Selektionsmarker steht unter der transkriptionellen Kontrolle von GAL4-Bindungsstellen im Rahmen unterschiedlicher Promotoren. Der Selektionsmarker URA3 wird durch den SPO13-Promotor reguliert, der aus dem Hefestamm MaV103 (vgl. Vidal, et al., Proc. Natl. Acad. Sci. USA (93), 10315-10320) stammt, ein negativ-regulatorisches Element enthält und durch starke Protein-Protein-Interaktionen aktiviert werden kann. Ferner enthält der Hefestamm das E.coli β-Galaktosidase-(-Gal)-Gen als weiteren Marker, dessen Aktivität leicht quantifizierbar ist und eine Abschätzung der in vivo Bindungsaffinität des Peptids an das E6-Protein ermöglicht. Auch die Aktivierung des HIS3-Gen kann durch Titration mit 3'AT-(3-Amino- -1, 2, 4-Triazol) quantifiziert werden.

Das HPV16 E6-Protein wird einem Screening mit vorstehendem System unterzogen. Hierzu wird es in Form eines es kodierenden Expressionsvektors bereitgestellt. Als Basisvektor dient pPC97 (vgl. Vidal et al. vorstehend), in dem die kodierende Sequenz eines HPV 16 E6-Proteins inseriert ist. Aus ca. 2 x 10⁶ Hefeklonen werden 15 Klone isoliert, die unter Selektion mit ADE2 ein Wachstum zeigen. Replika-Plattierungen und die Analyse der drei Selektionsmarker zeigen, daß 14 der 15 Klone auch unter Selektion mit HIS3 Wachstum zeigen (Fig. 2). Desweiteren zeigen 6 der isolierten Klone zusätzlich Wachstum unter URA3-Selektion, was unter den hier eingesetzten Bedingungen auf eine besonders hohe in vivo Affinität des entsprechenden Peptids für das HPV E6-Protein hinweist.

Zur weiteren Kontrolle werden aus den 15 Klonen die entsprechenden Peptid-Expressionsplasmide isoliert und nach erneuter Transformation in Hefe einem Rescreening unterzogen. Das Rescreening zeigt eine komplette Übereinstimmung mit den Ergebnissen aus den vorstehenden Replika-Plattierungen (Fig. 2). Es zeigt sich, daß das Wachstum der Hefe von der Bindung der Peptide an das HPV16 E6-Protein abhängt.

Die Peptide der 15 Klone werden in ihrer Aminosäuresequenz bestimmt. Diese ist in Tabelle 1, E61-1.pep - E61-17.pep angegeben. Ein Teil der Peptide zeigt Sequenzhomologieen zu den untereinander verwandten Bindungsdomänen der E6-bindenden Proteine E6-AP, E6-BP und Paxillin ("LhXΦLs-" ähnliche Motive, wobei: h = Q, E oder N; X = jede beliebige Aminosäure; Φ = hydrophobe Aminosäure; s = kleine Aminosäure wie G oder A und - = saure Aminosäure. Der andere Teil der E6-bindenden Peptide weist keine offensichtlichen Sequenz-Ähnlichkeiten zu den vorstehenden E6-bindenden Proteinen auf. Ihre Sequenzen können jedoch Hinweise auf bislang unbekannte E6-Interaktionspartner geben.

In einem weiteren Screening-Ansatz werden zwei weitere Klone erhalten. Ihre Aminosäuresequenz ist in Tabelle 1, E61-19.pep und E61-20.pep angegeben.

### Beispiel 2: Inhibierung der anti-apoptotischen Aktivität von HPV E6-Proteinen durch erfindungsgemäße Peptide.

Es wird das "VP 22-Shuttle-System" verwendet. Dieses beruht darauf, daß das HSV1-VP 22-Protein von Zellen aufgenommen wird, d.h. als Träger verwendet werden kann. Es werden Fusionspolypeptide aus VP 22 und erfindungsgemäßen Peptiden von Tabelle 1 hergestellt. Hierzu wird der Expressionsvektor pCEP4 (Invitrogen) verwendet. In diesen werden die DNA-Sequenzen der vorstehenden Peptide in Phase mit der DNA-Sequenz von VP 22 inseriert. Die Peptide werden dabei im Rahmen des E. Coli TrxA-Proteins exprimiert. Die erhaltenen Expressionsplasmide pCEP4/E61-1.pep bzw. pCEP4/E61-2.pep werden in HPV16 positive SiHa bzw. CaSKi-Zervixkarzinom-Zellen transfiziert. Zu verschiedenen Zeitpunkten wird die Morphologie der Zellen und ihr Wachstum durch übliche Verfahren untersucht.

Es zeigt sich, daß durch die erfindungsgemäßen Peptide die anti-apoptotische Aktivität von HPV E6-Proteinen inhibiert werden kann. Ferner zeigt sich, daß die durch HPV E7-Proteine induzierte Apoptose erhalten wird. Dies spiegelt sich auch in "Colony-Formation-Assays" wieder, die eine starke Wachstumsinhibition der Zellen zeigen. Somit eignet sich ein erfindungsgemäßes Peptid HPV-positive Zellen, insbesondere HPV-Tumorzellen, zu eliminieren.

## Patentansprüche

1. Peptid, ausgewählt aus den folgenden Peptiden
NH₂-GALVHKLFSQ TSGSCLVCIS-COOH
NH₂-LDVLGCLVRR LGVVLVGLH-COOH
NH₂-CYVECGCEVL TALVNGVRVL-COOH
NH₂-GVGGLCSCAS CVSEDFYASV-COOH
NH₂-IDLLRRLGSQ LHLLLVSVGG-COOH
NH₂-LAVLLNGYTRAIVGISFGGW-COOH
NH₂-LCTMCATVFR PLLVWFWSIW-COOH
NH₂-QLLLDLLLGS YEGMSLTSSP-COOH
NH₂-SRSNALHTLD VLLGGT-COOH
NH₂-GGAVYLCDAG CCFYCCGCSG-COOH
NH₂-CLELFDDLFL ALSLLLLVGG-COOH
NH₂-PLCRTCLIES AVLIQLSRL-COOH
NH₂-VFSGVYYAEF VFAASAGGTP-COOH
NH₂-MAPVGAGRPC CTVCFLTARF-COOH
NH₂-LSMLLFAAKL PVAVLCSWQA-COOH
NH₂-LVGRVRIGVS VFIRGGRLL-COOH
NH₂-LFDIFRLCAQPVLVHGHTRV-COOH
wobei das Peptid eine Modifikation in der Sequenz von bis zu 40 % aufweisen kann.

2. Peptid nach Anspruch 1, wobei das Peptid als Fusionspolypeptid vorliegt.

3. Peptid nach Anspruch 2, wobei das Fusionspolypeptid eine "Leader"-Sequenz umfaßt.

4. DNA, kodierend für das Peptid nach einem der Ansprüche 1 - 3.

5. Expressionsvektor, enthaltend die DNA nach Anspruch 4.

6. Antikörper, gerichtet gegen das Peptid nach Anspruch 1

7. Zusammensetzung, umfassend ein oder mehrere Peptide nach einem der Ansprüche 1 - 3, ein oder mehrere Expressionsvektoren nach Anspruch 5 und/oder einen oder mehrere Antikörper anch Anspruch 6 sowie übliche Hilfsstoffe.

8. Zusammensetzung nach Anspruch 7, wobei das Peptid als Fusionspolypeptid vorliegt.

9. Zusammensetzung nach Anspruch 8, wobei das Fusionspolypeptid eine "Leader"-Sequenz umfaßt.

10. Verwendung des Peptids nach einem der Ansprüche 1 - 3, des Expressionsvektors nach Anspruch 5 oder der Zusammensetzung nach einem der Ansprüche 7 - 9 für die Hestellung eines Medikamentes zur Inhibierung eines HPV E6-Proteins.

11. Verwendung nach Anspruch 10, wobei die Inhibierung eine Eliminierung von HPV-positiven Zellen umfaßt.

12. Verwendung nach Anspruch 11, wobei die HPV-positiven Zellen von einer HPV-assoziierten Erkrankung stammen.

13. Verwendung nach einem der Ansprüche 9 - 11, wobei die Inhibierung des HPV E6-Proteins zur Behandlung von HPV-assoziierten Erkrankungen erfolgt.

14. Verwendung nach Anspruch 12 oder 13, wobei die HPV-assoziierte Erkrankung eine HPV-Infektion, eine HPV-Dysplasie und ein HPV-Karzinom umfaßt.

15. Verwendung nach einem der Ansprüche 10-14, wobei HPV HPV1, HPV5, HPV6, HPV11, HPV16, HPV18, HPV 31, HPV 33 und HPV 35 umfaßt.

## Claims

1. A peptide selected from the following peptides
NH₂-GALVHKLFSQ TSGSCLVCIS-COOH
NH₂-LDVLGCLVRR LGWLVGLH-COOH
NH₂-CYVECGCEVL TALVNGVRVL-COOH
NH₂-GVGGLCSCAS CVSEDFYASV-COOH
NH₂-IDLLRRLSQ LHLLLVSVGG-COOH
NH2-LAVLLNGYTRAIVGISFGGW-COOH
NH₂-LCTMCATVFR PLLVWFWSIW-COOH
NH₂-QLLLDLLLGS YEGMSLTSSP-COOH
NH₂-SRSNALHTLD VLLGGT-COOH
NH₂-GGAVYLCDAG CCFYCCGCSG-COOH
NH₂-CLELFDDLFL ALSLLLLVGG-COOH
NH₂-PLCRTCLIES AVLIQLSRL-COOH
NH₂-VFSGVYYAEF VFAASAGGTP-COOH
NH₂-MAPVGAGRPC CTVCFLTARF-COOH
NH₂-LSMLLFAAKL PVAVLCSWQA-COOH
NH₂-LVGRVRIGVS VFIRGGRLL-COOH
NH₂-LFDIFRLCAQPVLVHGHTRV-COOH,
wherein the peptide may contain a sequence modification of up to 40 %.

2. The peptide according to claim 1, wherein the peptide is present as a fusion polypeptide.

3. The peptide according to claim 2, wherein the fusion polypeptide comprises a leader sequence.

4. A DNA coding for the peptide according to any one of claims 1 to 3.

5. An expression vector containing the DNA according to claim 4.

6. An antibody directed against the peptide according to claim 1.

7. A composition comprising one or several peptides according to any one of claims 1 to 3, one or several expression vectors according to claim 5 and/or one or several antibodies according to claim 6 as well as conventional auxiliary agents.

8. The composition according to claim 7, wherein the peptide is present as a fusion polypeptide.

9. The composition according to claim 8, wherein the fusion polypeptide comprises a leader sequence.

10. Use of the peptide according to any one of claims 1 to 3, the expression vector according to claim 5 or the composition according to any one of claims 7 to 9 for the manufacture of a medicament for inhibiting an HPV-E6 protein.

11. Use according to claim 10, wherein the inhibition comprises an elimination of HPV-positive cells.

12. Use according to claim 11, wherein the HPV-positive cells originate from an HPV-associated disease.

13. Use according to any one of claims 9 to 11, wherein the HPV-E6 protein is inhibited to treat HPV-associated diseases.

14. Use according to claim 12 or 13, wherein the HPV-associated disease comprises an HPV infection, an HPV dysplasia and an HPV carcinoma.

15. Use according to any one of claims 10 to 14, wherein HPV comprises HPV1, HPV5, HPV6, HPV11, HPV16, HPV18, HPV31, HPV33 and HPV35.

## Revendications

1. Peptide, choisi parmi les peptides suivants :
NH₂-GALVHKLFSQ TSGSCLVCIS-COOH
NH₂-LDVLGCLVRR LGVVLVGLH-COOH
NH₂-CYVECGCEVL TALVNGVRVL-COOH
NH₂-GVGGLCSCAS CVSEDFYASV-COOH
NH₂-IDLLRRLGSQ LHLLLVSVGG-COOH
NH₂-LAVLLNGYTRAIVGISFGGW-COOH
NH₂-LCTMCATVFR PLLVWFWSIW-COOH
NH₂-QLLLDLLLGS YEGMSLTSSP-COOH
NH₂-SRSNALHTLD VLLGGT-COOH
NH₂-GGAVYLCDAG CCFYCCGCSG-COOH
NH₂-CLELFDDLFL ALSLLLLVGG-COOH
NH₂-PLCRTCLIES AVLIQLSRL-COOH
NH₂-VFSGVYYAEF VFAASAGGTP-COOH
NH₂-MAPVGAGRPC CTVCFLTARF-COOH
NH₂-LSMLLFAAKL PVAVLCSWQA-COOH
NH₂-LVGRVRIGVS VFIRGGRLL-COOH
NH₂-LFDIFRLCAQPVLVHGHTRV-COOH
qui peut présenter dans la séquence une modification allant jusqu'à 40%.

2. Peptide suivant la revendication 1, qui se présente comme polypeptide de fusion.

3. Peptide suivant la revendication 2, pour lequel le polypeptide de fusion comprend une séquence « leader ».

4. ADN codant le peptide suivant l'une des revendications 1 à 3.

5. Vecteur d'expression, contenant l'ADN suivant la revendication 4.

6. Anticorps, dirigé contre le peptide suivant la revendication 1.

7. Composition comprenant un ou plusieurs peptides suivant l'une des revendications 1 à 3, un ou plusieurs vecteurs d'expression suivant la revendication 5 et/ou un ou plusieurs anticorps suivant la revendication 6 ainsi que des substances auxiliaires usuelles.

8. Composition suivant la revendication 7, dans laquelle le polypeptide est présent comme polypeptide de fusion.

9. Composition suivant la revendication 8, dans laquelle le polypeptide de fusion comprend une séquence « leader ».

10. Utilisation du peptide suivant l'une des revendications 1 à 3, du vecteur d'expression suivant la revendication 5 ou de la composition suivant l'une des revendications 7 à 9 pour la préparation d'un médicament destiné à inhiber la protéine E6 du virus HPV.

11. Utilisation suivant la revendication 10, dans laquelle l'inhibition comprend une élimination des cellules HPV-positives.

12. Utilisation suivant la revendication 11, dans laquelle les cellules HPV-positives proviennent d'une maladie associée au virus HPV.

13. Utilisation suivant l'une des revendications 9 à 11, dans laquelle l'inhibition de la protéine E6 du virus HPV est effectuée en vue du traitement de maladies associées au virus HPV.

14. Utilisation suivant la revendication 12 ou 13, dans laquelle la maladie associée au virus HPV est une infection, une dysplasie ou un carcinome associés au virus HPV.

15. Utilisation suivant l'une des revendications 10 à 14, dans laquelle le virus HPV comprend HPV1, HPV5, HPV6, HPV11, HPV16, HPV18, HPV 31, HPV 33 et HPV 35.
